# EUROPEAN PATENT APPLICATION

(11) **EP 4 681 702 A1**
(43) Date of publication of application: **21.01.2026**
(21) Application number: 24386086.3
(22) Date of filing: 16.07.2024
(51) Int. Cl.: A61K 9/00, A61K 9/08, A61K 31/495, A61K 47/10, A61K 47/40

(54) **ORAL SOLUTION COMPRISING VORTIOXETINE HYDROBROMIDE**

(71) Applicant: PharmaPath S.A., 12351 Agia Varvara (GR)
(72) Inventor: Kontopanou, Evanthia, 14561 Kifisia (GR); Vouvalidis, Ioannis, 14452 Metamorfosi (GR); Panagiotopoulos, Dimitrios-Tsampikos, 15124 Marousi (GR); Christodoulou, Eirini, 12462 Chaidari (GR)
(74) Representative: Roukounas, Dimitrios

(57) **Abstract**

An oral pharmaceutical solution comprising vortioxetine hydrobromide and a pharmaceutically acceptable aqueous carrier comprising hydroxypropylbetadex and a cosolvent system consisting of a mixture of ethanol and glycerol, wherein the pH of the solution is from 4.5 to 6.5.

## Description

### FIELD OF THE INVENTION

The present invention relates to oral pharmaceutical solutions comprising vortioxetine hydrobromide.

### BACKGROUND OF THE INVENTION

Vortioxetine, namely 1-[2-(2,4-dimethylphenylsulphanyl)phenyl]piperazine, is an atypical antidepressant that exerts a combined SERT-inhibitor, 5-HT3 antagonist and partial 5-HT1A agonist action. Vortioxetine as such is known from WO 03/029232 (example 1e).

Vortioxetine has the following structure.

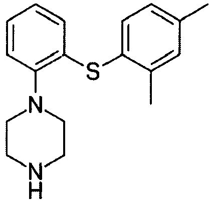

Vortioxetine is an antidepressant of the serotonin modulator and stimulator (SMS) class and used to treat major depression in adults. Major depression is a condition in which patients have mood disturbances that interfere with their everyday life. Symptoms often include deep sadness, feelings of worthlessness, loss of interest in favourite activities, sleep disturbances, a feeling of being slowed down, feelings of anxiety and changes in weight.

The solubility of vortioxetine in water is very low. The solubility can be increased by using various acids (such as hydrobromic acid, lactic acid, hydrochloric acid, methanesulfonic acid, fumaric acid, maleic acid, meso-tartaric acid, L-(+)-tartaric acid, L-(-)-tartaric acid, phosphoric acid, hydrochloric acid or nitric acid, etc.) and adjusting the pH to a suitable value.

Vortioxetine is present on the market under the trademark Brintellix^{®}. The finished product is presented as immediate-release film-coated tablets, containing 5, 10, 15 or 20mg of vortioxetine (as hydrobromide salt) as the active substance, and as oral drops containing 20mg/ml of vortioxetine (as the lactate salt) as the active substance.

Brintellix^{®} oral drops contains apart from vortioxetine DL-lactate, hydroxypropylbetadex, ethanol (96 %) and purified water. The oral drops can be taken with or without food. The drops can be mixed with water, juice or other non-alcoholic drinks
According to the European public assessment report (EPAR) of Brintellix^{®} (vortioxetine lactate) oral drops, which provides public information on how the European Medicines Agency (EMA) assessed Brintellix^{®} oral drops to recommend its authorisation in the EU, vortioxetine DL-lactate shows higher solubility in polar solvents than vortioxetine hydrobromide. For that reason, unlike the film-coated tablets where vortioxetine hydrobromide is used, the oral drops formulation contains vortioxetine DL-lactate as the active substance. The solubility of vortioxetine is further increased by using hydroxypropylbetadex in the formulation. The other excipient, ethanol 96%, increases the number of drops per ml and at the same time serves as preservative. A concentration of ethanol of 8.5% (w/v) gives the desired drop number of 20 drops/ml. The oral drops formulation is self-preserving which is confirmed with a test for efficacy of antimicrobial preservation. Purified water serves as vehicle for the formulation.

However, according to the "Guideline on pharmaceutical development of medicines for paediatric use" of the European Medicines Agency, oral drops can provide a useful means to administer medicinal products only in low doses or small volumes. Importantly, according to the guideline, there is a risk of counting the incorrect number of drops, and the accuracy and precision of the volume administered should always be justified in relation to the criticality of the dose. In order to avoid counting errors, alternative measuring devices should be considered where the dose comprises more than 10 drops. Apparently, this lack of convenience due to excessive number of drops required to deliver the required high doses of vortioxetine, may result in high incidence of non- compliance and ineffective therapy.

Thus, as a means of both ensuring flexibility in dose titration and advanced patient compliance, the development of oral pharmaceutical solutions which may also be administered by means of a device suitable for accurately measuring the prescribed volume, such as an oral syringe, a spoon or a cup, and which may also be administered without first diluting them with a liquid carrier, is definitely an existing need.

However, it is challenging to formulate vortioxetine oral solutions, which on the one hand are stable and on the other hand are palatable, since it is difficult to effectively taste mask the bad and very bitter taste of vortioxetine and its pharmaceutically acceptable salts, even at low concentrations.

CN104586756A discloses oral solutions containing vortioxetine hydrobromide and a solvent system comprising water, water miscible solvents, taste enhancing/masking agents, preservatives, and a pH adjusting agent, wherein the solution has a pH in the range of 3.0 to 5.0, preferably in the range 3.5 to 4.5. According to this application, the water-miscible solvent is selected from ethanol, glycerol, propylene glycol and sorbitol, and is preferably glycerol and/or propylene glycol. Example 1 discloses a composition comprising 6.355 mg/ml of vortioxetine hydrobromide, 324 mg/ml xylitol, 50 mg/ml glycerol, 25 mg/ml propylene glycol, citrate monohydrate, sodium citrate dihydrate, 0.5 mg/ml sodium benzoate, 0.5 mg/ml potassium sorbate, 1.0 mg/ml strawberry flavor and purified water.

CN104887624A describes oral solutions of vortioxetine comprising an acid-base regulator, such as bromic acid, lactic acid and hydrochloric acid, a co-solvent, such as mannitol, propylene glycol and polyethylene glycol 800, a flavouring agent, a preservative and water, wherein the solution has a pH in the range of 3.0 to 7.0.

The prior art indicates that high concentrations of taste masking agents such as sucrose, sugar alcohols and sweeteners, are necessary for masking the bitterness of vortioxetine. But even such high quantities are not sufficient to provide an effectively taste masked formulation. Thus, vortioxetine or its pharmaceutically acceptable salts are inevitably formulated in the form of oral drops that should be first diluted with a liquid carrier, such as water, juice or other non-alcoholic drinks prior to their administration.

The present invention addresses the problems of the prior art knowledge by advantageously providing a palatable and physicochemically stable oral pharmaceutical solution, which comprises vortioxetine hydrobromide and which can be administered without first diluting it with a liquid carrier or food.

### SUMMARY OF INVENTION

The present invention provides an oral pharmaceutical solution comprising vortioxetine hydrobromide as active ingredient.

The oral pharmaceutical solution according to the invention comprises vortioxetine hydrobromide and a pharmaceutically acceptable aqueous carrier comprising hydroxypropylbetadex and a cosolvent system consisting of a mixture of ethanol and glycerol, wherein the pH of the solution is from 4.5 to 6.5.

The oral pharmaceutical solution according to the invention exhibits excellent physicochemical stability, whereas at the same time it exhibits excellent organoleptic characteristics.

The oral pharmaceutical solution comprising vortioxetine hydrobromide according to the invention exhibits excellent taste masking effect while at the same time allows the optimal selection of concentration of taste masking excipients, surfactants or further cosolvents at the lowest feasible level. The use of these excipients at the lowest feasible level is very important, particularly in the case of formulations intended for paediatric population.

The oral pharmaceutical solution comprising vortioxetine hydrobromide according to the invention can be administered without having to be first diluted with a liquid carrier.

### DETAILED DESCRIPTION OF THE INVENTION

The present invention is directed to an oral pharmaceutical solution comprising vortioxetine hydrobromide in association with a pharmaceutically acceptable aqueous carrier.

In the prior art, attempts have been made to mitigate or reduce the bad and very bitter taste and aftertaste imparted by vortioxetine and its pharmaceutically acceptable salts when formulated as an oral liquid. These attempts include the use of taste masking agents such as sucrose, sugar alcohols and sweeteners in relatively high concentrations.

As shown in Example 1 of the present application, the use of high quantities of taste masking agents in the oral liquid formulations described in the prior art, such as in CN104586756A and CN104887624A, is not only insufficient to effectively taste mask the bitter taste and aftertaste of vortioxetine or its pharmaceutically acceptable salts, but it also results in compositions exhibiting an unpleasant sweetness. Furthermore, the relatively high concentrations of taste masking agents that are necessary for masking the bitterness of vortioxetine salts cause a negative effect on solubility, resulting in the formation of a suspension, with the presence of visible particles that become apparent upon production.

It has surprisingly been found that when vortioxetine hydrobromide is combined with an aqueous carrier comprising hydroxypropylbetadex and a cosolvent system consisting of ethanol and glycerol at a certain pH range, it is possible to manufacture an oral solution having excellent stability and improved organoleptic properties compared to compositions of the prior art. This finding is unexpected since it does not apply to other polyols, such as maltitol, sorbitol, xylitol, erythritol, isomalt and lactitol, as well as polyvinyl alcohol.

Furthermore, it has surprisingly been found that when vortioxetine hydrobromide is combined with an aqueous carrier comprising hydroxypropylbetadex and a cosolvent system consisting of a mixture of ethanol and glycerol at a certain pH range, it is possible to manufacture an oral solution, instead of a suspension, having improved organoleptic properties compared to compositions of the prior art, by using very low concentration of a sweetener.

Hydroxypropylbetadex (also known as β-Cyclodextrin, 2-hydroxypropyl ether) is a pharmaceutically acceptable excipient prepared by the treatment of an alkaline solution of β-cyclodextrin with propylene oxide. Hydroxypropylbetadex has been widely investigated in pharmaceuticals and has principally been used as a solubilizer for hydrophobic molecules in oral liquids. The reported advantage of hydroxypropylbetadex over unsubstituted b-cyclodextrin is its greater water solubility.

Thus, the present invention provides an oral pharmaceutical solution comprising vortioxetine hydrobromide and a pharmaceutically acceptable aqueous carrier comprising hydroxypropyl betadex and a cosolvent system consisting of a mixture of ethanol and glycerol, wherein the pH of the solution is from 4.5 to 6.5.

Preferably, the oral pharmaceutical solution according to the invention comprises from 1 mg/ml to 30 mg/ml vortioxetine hydrobromide. More preferably, the oral pharmaceutical solution according to the invention comprises from 2 mg/ml to 20 mg/ml vortioxetine hydrobromide. Even more preferably, the oral pharmaceutical solution according to the invention comprises from 3 mg/ml to 10 mg/ml vortioxetine hydrobromide.

Preferably, the concentration of hydroxypropylbetadex in the oral solution is from 1 mg/ml to 300 mg/ml, more preferably, from 5 mg/ml to 200 mg/ml and even more preferably, from 10 mg/ml to 100 mg/ml.

Preferably, the weight ratio of vortioxetine hydrobromide to hydroxypropylbetadex in the oral solution is from 1:1 to 1:20, more preferably from 1:3. to 1:13 and even more preferably, from 1:6 to 1:10.

Preferably, the concentration of ethanol in the oral solution is from 5 mg/ml to 65 mg/ml, more preferably, from 10 mg/ml to 60 mg/ml and even more preferably, from 15 mg/ml to 55 mg/ml.

Preferably, the concentration of glycerol in the oral solution is from 10 mg/ml to 160 mg/ml, more preferably, from 20 mg/ml to 140 mg/ml and even more preferably, from 30 mg/ml to 120 mg/ml.

Preferably, weight ratio of ethanol to glycerol in the oral solution is from 1:0.5 to 1:4, more preferably from 1:0.6 to 1:3.5 and even more preferably, from 1:0.7 to 1:3.

Preferably, the pH of the solution is from 5.0 to 6.0.

Preferably, the oral pharmaceutical solution according to the invention is free of a sugar alcohol, such as, maltitol, mannitol, sorbitol, xylitol, erythritol, isomalt and lactitol, as well as polyvinyl alcohol.

According to a preferred embodiment, the oral pharmaceutical solution according to the invention comprises
from 1 mg/ml to 30 mg/ml of vortioxetine hydrobromide, and
a pharmaceutically acceptable aqueous carrier comprising
   from 1 mg/ml to 300 mg/ml hydroxypropylbetadex, and a cosolvent system consisting of a mixture of ethanol and glycerol,
   wherein the concentration of ethanol in the solution is from 5 mg/ml to 65 mg/ml. wherein the concentration of glycerol in the solution is from 10 mg/ml to 160 mg/ml,
and wherein the pH of the solution is from 4.5 to 6.5.

According to another preferred embodiment, the oral pharmaceutical solution according to the invention comprises
from 3 mg/ml to 10 mg/ml of vortioxetine hydrobromide, and
a pharmaceutically acceptable aqueous carrier comprising
   from 10 mg/ml to 100 mg/ml hydroxypropylbetadex, and a cosolvent consisting of a mixture of ethanol and glycerol,
   wherein the concentration of ethanol in the solution is from 15 mg/ml to 55 mg/ml. wherein the concentration of glycerol in the solution is from 30 mg/ml to 120 mg/ml,
wherein the weight ratio of vortioxetine hydrobromide to hydroxypropylbetadex in the solution is from 1:6 to 1:10,
wherein the weight ratio of ethanol to glycerol in the solution is from 1:0.7 to 1:3, and wherein the pH of the solution is from 5.0 to 6.0.

The oral pharmaceutical solution according to the invention may also comprise additional excipients commonly used in preparing oral liquid compositions, such as buffering agents, antimicrobial preservatives, antioxidants, sweeteners and flavouring agents.

Any pharmaceutically acceptable system which acts as a buffer in the pH region of the invention can be used in the oral pharmaceutical solution. Buffering agents may include but not limited to ascorbic acid, acetic acid, tartaric acid, citric acid monohydrate, sodium citrate, potassium citrate, acetic acid, sodium acetate, sodium hydrogen phosphate, sodium dihydrogen phosphate, calcium hydrogen phosphate, calcium dihydrogen phosphate or mixtures thereof.

Antimicrobial preservatives may include but are not limited to sodium benzoate, benzoic acid, boric acid, sorbic acid and their salts thereof, benzyl alcohol, parahydroxybenzoic acids and their alkyl esters, methyl, ethyl and propyl parahydroxybenzoates and their salts, or mixtures thereof.

Antioxidants which may be used in the present invention comprise, amongst others, butylated hydroxytoluene, butylated hydroxyanisole, ethylenediamine tetraacetic acid ("EDTA"), ascorbic acid, sodium metabisulfite and propyl gallate, or mixtures thereof.

Sweeteners may include but are not limited to sucralose, aspartame, acesulfame potassium, thaumatin, saccharin and salts thereof, sodium cyclamate, glycyrrhizin, monosodium glycyrrhizinate, monoamonium glycyrrhizinate, or mixtures thereof.

The oral pharmaceutical solution according to the invention may further comprise flavours and/or colours so as to enhance its palatability and/or visual appearance. Suitable flavouring agents and colouring agents are well known to those skilled in the art. The flavouring agent may be a natural or artificial flavouring agent, including an essence, an extract, a flavour oil, or combinations thereof. Exemplary flavours include, but are not limited to honey flavour, raspberry flavour, strawberry flavour, blueberry flavour, blackberry flavour, grape flavour, peach flavour, apricot flavour, watermelon flavour, melon flavour, fruit punch flavour, cranberry flavour, mango flavour, banana flavour, citrus flavour, orange flavour, lemon flavour, grapefruit flavour, cherry flavour, vanilla flavour, caramel flavour, chocolate flavour, marshmallow flavour, coffee flavour and coconut flavour.

The oral pharmaceutical solution according to the invention is preferably supplied as multidose preparation. Each dose from a multidose container may be administered by means of a device suitable for accurately measuring the prescribed volume. The device is usually a spoon or a cup for volumes of 5 mL or multiples thereof, or an oral syringe for other volumes. Preferably, the device is an oral syringe.

The oral pharmaceutical solution of the present invention may be prepared using methods well known in the art and using regular manufacturing equipment.

For example, it may be prepared using the following process: Vortioxetine hydrobromide and the excipients are weighed. A quantity of purified water is added into a vessel. Hydroxypropylbetadex is then added into a vessel under stirring until it is totally dissolved. Ethanol and glycerol are then added into the vessel under stirring until they are totally dissolved. The remaining excipients, if present, are successively added under continuous stirring, until they are totally dissolved. Finally, the volume is adjusted with a quantity of purified water.

The final solution is optionally filtered over a 10 µm filter, and filled preferably in light-protective containers, such as amber type III glass 50- or 100-ml bottles sealed with child resistant, tamper evident screw caps.

The oral pharmaceutical solution of the present invention exhibits excellent organoleptic properties. This means that the solution can be administered orally to a patient without prior dilution with a liquid carrier. Thus, the present invention provides also an oral pharmaceutical solution as described herein for use in a method of treatment of depression in a human patient, wherein the method comprises administering orally the solution to the patient without diluting the solution with a liquid carrier prior to the administration.

### EXAMPLES

### EXAMPLE 1

The example shows the improved solubility/stability and palatability of a solution according to the present invention compared to the solubility/stability and palatability of a solution according to the prior art (Example 1 of CN104586756 A). The compositions of the solutions are shown in Table 1 below.

Trial 1 (solution according to the invention) was prepared in the following manner:
In a vessel about 80% of the total amount of purified water was added and set under stirring. Gradually hydroxypropylbetadex was added under continuous stirring. In the same vessel, vortioxetine hydrobromide was added under continuous stirring. Ethanol and glycerol were then added under continuous stirring. The remaining excipients were added under continuous stirring. The pH was then adjusted with HCl or NaOH 0.1N at the desired pH level. Finally, the volume was adjusted with purified water.

Trial 2, (Ex. 1 CN104586756 A) was prepared in the following manner:
In a vessel about 80% of the total amount of purified water was added and set under stirring. Gradually glycerol and propylene glycol were added under continuous stirring. In the same vessel, vortioxetine hydrobromide was added under continuous stirring. Then, xylitol, sodium benzoate, potassium sorbate, citric acid monohydrate and sodium citrate dihydrate were added in the vessel under continuous stirring. The remaining excipients were added under continuous stirring. The pH was then adjusted with HCl or NaOH 0.1N at the desired pH level. Finally, the volume was adjusted with purified water.

**Table 1**

| **Component** | **Function** | **Vortioxetine oral solution (5 mg/mL)** | **Ex. 1** CN104586756 A |
|---|---|---|---|
| | | **Trial 1** | **Trial 2** |
| | | mg/ml | |
| Vortioxetine HBr | API | 6.355 | 6.355 |
| Hydroxypropylbetadex | Solubility enhancer | 50 | - |
| Ethanol | Co-Solvent(s) | 50 | - |
| Glycerol | | 50 | 50 |
| Propylene glycol | | - | 25 |
| Citric acid monohydrate | Buffering agent | - | 1.64 |
| Sodium citrate dihydrate | | - | 1.0 |
| Sodium benzoate | Preservatives | - | 0.5 |
| Potassium sorbate | | - | 0.5 |
| Sucralose | Sweetener | 0.5 | - |
| Xylitol | | - | 324 |
| Strawberry flavor | Flavour | 1.0 | 1.0 |
| NaOH/ HCl sol. 0.1% | pH adjusting agent | Qs to pH 5.5 | Qs to pH 4.0 |
| Purified water | Solvent | Qs to 1 mL | Qs to 1 mL |
| Flavour score (mean scores of five judges) | | 8.6 | 4.4 |

The composition of Trial 2 was found to be unacceptable resulting in the formation of a suspension, with the presence of visible particles that become apparent during production.

The compositions of Trial 1 & 2 were also studied in relation to their aftertaste after oral administration. The flavor test results were based on the analysis of five trained judges who are experienced in detailed flavor analysis. The compositions were administered by means of a syringe and they were administered directly into the mouth and swallowed i.e. without diluting them with a liquid carrier prior to their administration. 2 ml of composition were administered each time. The scoring system includes a scale of 1-10, wherein 1 designates the most unpleasant taste and 10 to the most pleasant taste. The flavour scores are shown in Table 1.

Five out of five judges preferred the composition of Trial 1 to the composition of Trial 2 since they all noticed that the bitter aftertaste was more intense in case of Trial 2, while they all noticed an unpleasant extreme sweetness when solution came into contact with the tongue.

The compositions of Trial 1 & 2 were also stored at a temperature of 40°C and a relative humidity of 75% for a total period of three months. Quantification of vortioxetine hydrobromide and its impurities in the compositions was performed by HPLC. The results of the stability study are shown in Table 2.

**Table 2: Stability results**

| | **Trial 1** | **Trial 2** |
|---|---|---|
| T = 0 results | | |
| Appearance | clear colourless solution | precipitation was observed within minutes of the preparation of the composition |
| Assay | 98.9% | < 90% |
| Max Unknown Impurity | Not detected | Not performed |
| Total impurities | Not detected | |
| Stability T = 3 months (40 °C/75% RH) | | |
| Assay | 98.5% | Not performed |
| Max Unknown Imp | RRT 1.3 <0.05% RRT 0.85 <0.05% | |
| Total impurities | <0.05% | |

| | | |
|---|---|---|
| RRT: Relative Retention Time | | |

In Table 2, the term "maximum unknown impurity" refers to an unknown (not specified) impurity of vortioxetine hydrobromide, which in the chromatogram gives the greater peak and thus corresponds to the greater impurity concentration in the solution, while "total impurities" is the sum of all known (specified) and unknown (not specified) impurities of vortioxetine hydrobromide observed in the chromatogram.

### EXAMPLE 2

The example shows the improved palatability of a solution according to the present invention compared to the palatability of solutions according to the prior art (Vortioxetine oral solution based on Brintellix^{®} oral drops as Trial B & commercially available Brintellix^{®} oral drops). The compositions of the solutions are shown in Table 3 below.

Trials 3 & A were prepared in the following manner:
In a vessel about 80% of the total amount of purified water was added and set under stirring. Gradually hydroxypropylbetadex was added under continuous stirring. In the same vessel, vortioxetine hydrobromide was added under continuous stirring. Ethanol (trials 3 & A) and glycerol (trial 3) were then added under continuous stirring. The pH was then adjusted with HCl or NaOH 0.1N at the desired pH level. Finally, the volume was adjusted with purified water.

The quantitative composition of commercial "Brintellix^{®} oral drops, solution" product was approached by a set of laboratory studies, including complexation studies for assessing the concentration of hydroxypropylbetadex. Information regarding concentration of ethanol is publicly available through the SmPC of the product.

**Table 3**

| **Component** | **Function** | **Vortioxetine oral solution** | | **Brintellix^{®} oral drops** |
|---|---|---|---|---|
| | | **Trial 3** | **Trial A** | |
| | | mg/ml | | |
| Vortioxetine HBr | API | 25.42 | 25.42 | - |
| Vortioxetine lactate | | - | | 26.04 |
| Hydroxypropylbetadex | Solubility enhancer | 200 | 200 | 200 |
| Ethanol | Co-Solvent(s) | 50 | 85 | 85 |
| Glycerol | | 35 | - | - |
| NaOH/ HCl sol. 0.1% | pH adjusting agent | Qs to pH 5.0 | | pH 5.1 |
| Purified water | Solvent | Qs to 1 mL | | - |
| Flavour score (mean scores of five judges) | | 6.7 | 3.5 | 3.7 |

The solutions of Trial 3 & A as well as Brintellix^{®} oral drops were studied in relation to their aftertaste after their oral administration. The flavor test results were based on the analysis of five trained judges who are experienced in detailed flavor analysis. All solutions were administered by means of a syringe and they were administered directly into the mouth and swallowed i.e. without diluting them with a liquid carrier prior to their administration. 1 ml of solution was administered each time. The scoring system includes a scale of 1-10, wherein 1 designates the most unpleasant taste and 10 to the most pleasant taste. The flavour scores are shown in Table 3.

Five out of five judges preferred the solution of Trial 3 to solution of Trial A since they all noticed that the bitter aftertaste was more intense in case of Trial A. The bitter aftertaste in case of Brintellix^{®} oral drops was found to be totally unacceptable by all judges.

The solution of Trial 3 was also stored at a temperature of 40°C and a relative humidity of 75% for a total period of three months. Quantification of vortioxetine hydrobromide and its impurities in the compositions was performed by HPLC. The results of the stability study are shown in Table 4.

**Table 4: Stability results**

| | **Trial 3** |
|---|---|
| T = 0 results | |
| Appearance | clear colourless solution |
| Assay | 99.4% |
| Max Unknown Impurity | Not detected |
| Total impurities | Not detected |

| Stability T = 3 months (40C/75%RH) | |
|---|---|
| Assay | 100.7% |
| Max Unknown Impurity | RRT 1.3 <0.05% RRT 0.85 <0.05% |
| Total impurities | <0.05% |

### EXAMPLE 3

The example shows the improved palatability of a solution according to the present invention compared to the palatability of solutions according to the prior art (Brintellix^{®} oral drops). The compositions of the solutions are shown in Table 5 below.

Trial 4 was prepared using a process similar to the process used in case of Trial 3.

**Table 5**

| **Component** | **Function** | **Vortioxetine oral solution** | **Brintellix^{®} oral drops** |
|---|---|---|---|
| | | **Trial 4** | |
| | | mg/ml | |
| Vortioxetine HBr | API | 6.355 | - |
| Vortioxetine lactate | | - | 26.04 |
| Hydroxypropylbetadex | Solubility enhancer | 50 | 200 |
| Ethanol | Co-Solvent(s) | 40 | 85 |
| Glycerol | | 45 | - |
| NaOH/ HCl sol. 0.1% | pH adjusting agent | Qs to pH 5.0 | pH 5.1 |
| Purified water | Solvent | Qs to 1 mL | - |
| Flavour score (mean values) | | 6.5 | 5.2 |

The solutions of Trial 4 & Brintellix^{®} oral drops were studied in relation to their aftertaste after their oral administration.

Before testing, 5 ml of Brintellix^{®} oral drops were diluted in 15 ml of water (final concentration of vortioxetine lactate: 6.51 mg/ml).

The flavor test results were based on the analysis of five trained judges who are experienced in detailed flavor analysis. All solutions were administered by means of a syringe and they were administered directly into mouth and swallowed i.e. without diluting them with a liquid carrier prior to their administration. 2 ml of solution were administered each time. The scoring system includes a scale 1-10, wherein 1 designates the most unpleasant taste and 10 to the most pleasant taste. The flavour scores are shown in Table 5.

Five out of five judges preferred the solution of Trial 4 to diluted Brintellix^{®} oral drops since they all noticed that the bitter aftertaste was more intense in case of Brintellix^{®} oral drops.

### EXAMPLE 4

The example shows the improved palatability of a solution according to the present invention comprising a cosolvent system consisting of a mixture of ethanol and glycerol compared to the palatability of solutions comprising other polyols, such as mannitol or sorbitol. The compositions of the solutions are shown in Table 6 below.

Trials 5, B & C were prepared in the following manner:
In a vessel about 80% of the total amount of purified water was added and set under stirring. Gradually hydroxypropylbetadex was added under continuous stirring. In the same vessel, vortioxetine hydrobromide was added under continuous stirring. Ethanol (trials 5 & B), glycerol (trials 5 & C), mannitol (trial B) or sorbitol (trial C) were then added under continuous stirring. Sucralose was then added under continuous stirring. The pH was then adjusted with HCl or NaOH 0.1N at the desired pH level. Finally, the volume was adjusted with purified water.

**Table 6**

| **Component** | **Function** | **Vortioxetine oral solution** | | |
|---|---|---|---|---|
| | | **Trial 5** | **Trial B** | **Trial C** |
| | | mg/ml | | |
| Vortioxetine HBr | API | 6.355 | 6.355 | 6.355 |
| Hydroxypropylbetadex | Solubility enhancer | 50 | 50 | 50 |
| Ethanol | Co-Solvent(s) | 50 | 50 | - |
| Glycerol | | 100 | - | 100 |
| Mannitol | | - | 100 | 50 |
| Sorbitol | | - | - | 50 |
| Sucralose | Sweetener | 1.0 | 1.0 | 1.0 |
| NaOH/ HCl sol. 0.1% | pH adjusting agent | Qs to pH 5.0 | Qs to pH 5.0 | Qs to pH 5.0 |
| Purified water | Solvent | Qs to 1 mL | Qs to 1 mL | Qs to 1 mL |
| Flavour score (mean scores of five judges) | | 9.3 | 5.4 | 6.7 |

The solutions of Trial 5, B & C were studied in relation to their aftertaste after their oral administration. The flavor test results were based on the analysis of five trained judges who are experienced in detailed flavor analysis. All solutions were administered by means of a syringe and they were administered directly into the mouth and swallowed i.e. without diluting them with a liquid carrier prior to their administration. 1 ml of solution was administered each time. The flavour scores are shown in Table 6.

Five out of five judges preferred the solution of Trial 4 to solution of Trial B & Trial C since they all noticed that the bitter aftertaste was more intense in case of Trial B & Trial C.

The solution of Trial 5 was also stored at a temperature of 40°C and a relative humidity of 75% for a total period of three months. Quantification of vortioxetine hydrobromide and its impurities in the compositions was performed by HPLC. The stability results are shown in Table 7.

**Table 7: Stability results**

| | **Trial 5** |
|---|---|
| T = 0 results | |
| Appearance | clear colourless solution |
| Assay | 99.1% |
| Max Unknown Impurity | Not detected |
| Total impurities | Not detected |

| Stability T = 3 months (40C/75%RH) | |
|---|---|
| Assay | 99.7% |
| Max Unknown Impurity | RRT 1.3 <0.05% RRT 0.85 <0.05% |
| Total impurities | <0.05% |

## Claims

1. An oral pharmaceutical solution comprising vortioxetine hydrobromide and a pharmaceutically acceptable aqueous carrier comprising hydroxypropylbetadex and a cosolvent system consisting of a mixture of ethanol and glycerol, wherein the pH of the solution is from 4.5 to 6.5.

2. The oral pharmaceutical solution according to claim 1, wherein the concentration of vortioxetine hydrobromide in the solution is from 1 mg/ml to 30 mg/ml.

3. The oral pharmaceutical solution according to any one of the preceding claims, wherein the concentration of vortioxetine hydrobromide in the solution is from 2 mg/ml to 20 mg/ml.

4. The oral pharmaceutical solution according to any one of the preceding claims, wherein the concentration of vortioxetine hydrobromide in the solution is from 3 mg/ml to 10 mg/ml.

5. The oral pharmaceutical solution according to any one of the preceding claims, wherein the concentration of hydroxypropylbetadex in the solution is from 1 mg/ml to 300 mg/ml.

6. The oral pharmaceutical solution according to any one of the preceding claims, wherein the concentration of hydroxypropylbetadex in the solution is from 5 mg/ml to 200 mg/ml.

7. The oral pharmaceutical solution according to any one of the preceding claims, wherein the concentration of hydroxypropylbetadex in the solution is from 10 mg/ml to 100 mg/ml.

8. The oral pharmaceutical solution according to any one of the preceding claims, wherein the weight ratio of vortioxetine hydrobromide to hydroxypropylbetadex in the solution is from 1:1 to 1:20.

9. The oral pharmaceutical solution according to any one of the preceding claims, wherein the concentration of ethanol in the solution is from 5 mg/ml to 65 mg/ml.

10. The oral pharmaceutical solution according to any one of the preceding claims, wherein the concentration of glycerol in the solution is from 10 mg/ml to 160 mg/ml.

11. The oral pharmaceutical solution according to any one of the preceding claims, wherein the pH of the solution is from 5.0 to 6.0.

12. The oral pharmaceutical solution according to any one of the preceding claims, wherein the weight ratio of ethanol to glycerol in the solution is from 1:0.5 to 1:4.

13. The oral pharmaceutical solution according to any one of the preceding claims, wherein the solution further comprises a sweetener.

14. The oral pharmaceutical solution according to any one of the preceding claims, wherein the solution is free of a sugar alcohol and polyvinyl alcohol.

15. The oral pharmaceutical solution according to any one of the preceding claims for use in a method of treatment of depression in a human patient, wherein the method comprises administering orally the solution to the patient without diluting the solution with a liquid carrier prior to the administration.
